# EUROPEAN PATENT APPLICATION

(11) **EP 1 970 440 A1**
(43) Date of publication of application: **17.09.2008**
(21) Application number: 07103648.7
(22) Date of filing: 06.03.2007
(51) Int. Cl.: C12N 9/10

(54) **Polymerase stabilization by ionic detergents**

(71) Applicant: Qiagen GmbH, 40724 Hilden (DE)
(72) Inventor: Fang, Nan, 41468 Neuss (DE); Löffert, Dirk, 40724 Hilden (DE); Erbach, Christoph, 42781 Haan (DE); Peters, Lars-Erik, Lafayette, CO 80026 (US)
(74) Representative: Vossius & Partner

(57) **Abstract**

The invention relates to a composition comprising (i) an enzyme with nucleic acid polymerase activity, (ii) an inert protein and, (ii) an ionic detergent. The invention relates also to a composition comprising (i) an enzyme with nucleic acid polymerase activity, (ii) an inert protein and, (ii) a zwitterionic detergent. The invention further relates to a method for enzymatic nucleic acid synthesis comprising the steps of, (a) providing in a reaction mixture, a polymerase activity, a nucleic acid template, a ionic detergent, preferably zwitterionic detergent, a buffer, a salt, nucleotides and an inert protein and, (b) incubating the reaction mixture at a temperature which enables nucleic acid synthesis.

## Description

### Field of the invention

The present invention relates to protein stabilization, particularly the stabilization of polymerases in aqueous solutions containing ionic, particularly zwitterionic detergents and an inert protein.

### Background of the invention

Stabilization of enzymes is necessary for the long term storage and utilization in many biochemical and biotechnological processes. Enzymes have been isolated from thermophilic organisms which are stable to denaturation by heat. However, even these highly thermostable enzymes may be inactivated by chemical agents, proteases or environmental modifications. The purification and/or utilization of thermostable and other enzymes often requires concomitant use of denaturing conditions including highly elevated temperatures, aqueous environments with sub-optimal concentrations of co-factors and substrates, and a pH that is sub-optimal for maximum enzyme stability.

Many stabilization techniques are known. These techniques include immobilization of the enzyme on solid substrates, chemical modification of the enzyme, genetic engineering of the enzyme and the addition of stabilizing additives. Surfactants are one group of additives that have been shown to stabilize enzymes. Surfactants also called detergents are surface active compounds that stabilize the interface between the active form of the enzyme and the liquid environment in which they are contained.

For example, US patent 6,242,235 B1 disclose polymerase stabilization by polyethoxylated amine surfactants. Also disclosed therein are cationic surfactants for the stabilization of polymerases.

Non-ionic detergents have been variously shown to increase the solution stability of various proteins with enzymatic activity (e.g. cAMP-dependent protein kinase, tyrosine hydroxylase, nitric oxide synthase, tryptophane hydroxylase and a sweet potatoe beta-amylase).

Additionally non-ionic detergents such as TRITON X-100 and Tween 20 have been shown to stabilize the activity of DNA polymerases (Biochem. 14: 789-95, 1975).

European Patent Application 776 970 A1, incorporated herein by reference, discloses the use of non-ionic detergents including polyethoxylated sorbitan monolaurat (Tween 20) and ethoxylated alkyl phenol (NP-40) to stabilize the activity of thermostable Taq DNA polymerase.

Low concentrations of the anionic detergents sodium dodecyl sulphate (SDS) have been shown to stabilize enzyme activity. However, due to the possibility of cooperative binding, if the optimal concentration of SDS is exceeded in solution, the use of SDS in protein stabilization is limited. It is known, however, that many cationic detergents bind less strongly to proteins than strong anionic detergents such as SDS (Nozaki et al., J. Biol. Chem. 249: 4452-59, 1974).

Furthermore, most proteins have fewer cationic binding sites than anionic binding sites.

US 6,787,305 B1 discloses nitrogen-containing organic compounds, preferably 4-methylmorpholine N-oxid or betaine (carboxymethyltrimethylammonium) as enzyme stabilzers. The reactions disclosed in US 6,787,305 B1 may further comprise one or more compounds selected from the group consisting of proline and an N-alkylimidazole compound, and more preferably proline, 1-methyliimidazole or 4-methylimidazole.

WO 99/67371 discloses enzyme stabilization by cationic surfactants. In particular a polyethoxylated amine is disclosed.

US 2006/0035360 relates to methods and compositions for providing purified thermostable enzymes, particularly thermostable DNA polymerases that are free of exogenous detergents. This application also discloses the addition of one or more detergents selected from the group consisting of Tween 20, Iconol NP-40, Mega-8, Mega-9, Mega-10, alkyl glycosides, and alkyl tertiary amine N-oxides.

Said alkyl glycosides may be selected from octyl-beta-D-glucopyranoside and dodecyl-beta-D-maltoside.

US 6,127,155 relates to the stabilization of thermostable nucleic acid polymermases by making use of compositions containing non-ionic polymeric detergents.

The drawback with the art as described above, is in various cases a (i) high denaturing effect, (ii) a positive or negative charge, (iii) a low efficiency in disrupting aggregation and (iv) an often difficult removal of the detergent after the performance of the reaction.

Thus, there is a need for methods and compositions comprising one or more detergents for stabilizing a polymerase, which have, e.g. a low denaturing effect, no charge, a high efficiency in disrupting aggregation and/or, wherein the detergent involved is easily removed after the reaction.

### Description of the invention

The present invention relates to compositions and methods for stabilizing enzymes in particular polymerases. The inventors have astonishingly found that a composition comprising (a) an enzyme with nucleic acid polymerase activity, (b) an inert protein and, (c) an ionic detergent is an ideal stabilizer for enzymes in particular polymerases.
An inert protein refers to a natural occuring or synthetic peptide or polypeptide or mixtures thereof that does not interfer with the enzyme activity or enzyme reaction in question. Examples not limiting the scope of the present invention are globulin, albumin, collagen and derivatives thereof.
An enzyme with nucleic acid polymerase activity refers to the ability of an enzyme to synthesize nucleic acid strands (e.g., RNA or DNA) from ribonucleoside triphosphates or deoxynucleoside triphosphates. DNA polymerases synthesize DNA, while RNA polymerases synthesize RNA.

As used herein, the term "enzyme" refers to molecules or molecule aggregates that are responsible for catalyzing chemical and biological reactions. Such molecules are typically proteins, but can also comprise short peptides, RNAs, ribozymes, antibodies, and other molecules. A molecule that catalyzes chemical and biological reactions is referred to as "having enzyme activity" or "having catalytic activity."

As used herein, the terms "stabilization," "stabilizing," and "stabilized," when used in reference to enzyme activity refer to the ability of a material to maintain, enhance, or otherwise inhibit the decline or loss of the activity of an enzyme, often as measured over time (*i.e.,* in the presence of a stabilizer, an enzyme retains its activity for a longer time period than the enzyme in the absence of the stabilizer). "Stabilization of enzyme activity" also refers to the ability of a material to maintain the activity of an enzyme under suboptimal conditions of temperature or pH. As another example, "stabilizing enzyme activity" refers to the ability of a material to enhance enzyme activity under suboptimal conditions, as compared to activity in the absence of a "stabilizing" compound or material.

The term "polymerase" refers to an enzyme that synthesizes nucleic acid stands (e.g., RNA or DNA) from ribonucleoside triphosphates or deoxynucleoside triphosphates.

A variety of polypeptides having polymerase activity are useful in accordance with the present invention. Included among these polypeptides are enzymes such as nucleic acid polymerases (including DNA polymerases and RNA polymerases). Such polymerases include, but are not limited to, *Thermus thermophilus* (Tth) DNA polymerase, *Thermus aquaticus* (Taq) DNA polymerase, *Thermotoga neopolitana* (Tne) DNA polymerase, *Thermotoga maritima* (Tma) DNA polymerase, *Thermococcus litoralis* (Tli or VENT.TM.) DNA polymerase, *Pyrococcus furiosus* (Pfu) DNA polymerase, DEEPVENT. DNA polymerase, *Pyrococcus woosii* (Pwo) DNA polymerase, *Pyrococcus sp* KDD2 (KOD) DNA polymerase, *Bacillus sterothermophilus* (Bst) DNA polymerase, *Bacillus caldophilus* (Bca) DNA polymerase, *Sulfolobus acidocaldarius* (Sac) DNA polymerase, *Thermoplasma acidophilum* (Tac) DNA polymerase, *Thermus flavus* (Tfl/Tub) DNA polymerase, *Thermus ruber* (Tru) DNA polymerase, *Thermus brockianus* (DYNAZYME) DNA polymerase, *Methanobacterium thermoautotrophicum* (Mth) DNA polymerase, mycobacterium DNA polymerase (Mtb, Mlep), and mutants, variants and derivatives thereof. RNA polymerases such as T3, T5 and SP6 and mutants, variants and derivatives thereof may also be used in accordance with the invention. A preferred DNA polymerase is *Thermus eggertssonii* (Teg).

The nucleic acid polymerases used in the present invention may be mesophilic or therinophilic, and are preferably thermophilic. Preferred mesophilic DNA polymerases include T7 DNA polymerase, T5 DNA polymerase, Klenow fragment DNA polymerase, DNA polymerase III and the like. Preferred thermostable DNA polymerases that may be used in the methods and compositions of the invention include Teg, Taq, Tne, Tma, Pfu, Tfl, Tth, Stoffel fragment, VENT. and DEEPVENT DNA polymerases, and mutants, variants and derivatives thereof (U.S. Pat. No. 5,436,149; U.S. Pat. No. 4,889,818; U.S. Pat. No. 4,965,188; U.S. Pat. No. 5,079,352; U.S. Pat. No. 5,614,365; U.S. Pat. No. 5,374,553; U.S. Pat. No. 5,270,179; U.S. Pat. No. 5,047,342; U.S. Pat. No. 5,512,462; WO 92/06188; WO 92/06200; WO 96110640; Barnes, W. M., Gene 112:29-35 (1992); Lawyer, F. C., et al., PCR Meth. Appl. 2:275-287 (1993); Flaman, J.-M, et al., Nucl. Acids Res. 22(15):3259-3260 (1994)). For amplification of long nucleic acid molecules (e.g., nucleic acid molecules longer than about 3-5 Kb in length), at least two DNA polymerases (one substantially lacking 3' exonuclease activity and the other having 3' exonuclease activity) are typically used. See U.S. Pat. No. 5,436,149; U.S. Pat. No. 5,512,462; Barnes, W. M., Gene 112:29-35 (1992), and copending U.S. patent application Ser. No. 081801,720, filed Feb. 14, 1997, the disclosures of which are incorporated herein in their entireties. Examples of DNA polymerases substantially lacking in 3' exonuclease activity include, but are not limited to, Taq, Tne^{exo-}, Tma^{exo-}, Pfu^{exo-}, Pwo^{exo-} and Tth DNA polymerases, and mutants, variants and derivatives thereof.

Polypeptides having reverse transcriptase activity for use in the invention include any polypeptide having reverse transcriptase activity. Such enzymes include, but are not limited to, retroviral reverse transcriptase, retrotransposon reverse transcriptase, hepatitis B reverse transcriptase, cauliflower mosaic virus reverse transcriptase, bacterial reverse transcriptase, Tth DNA polymerase, Taq DNA polymerase (Saiki, R. K., et al., Science 239:487-491 (1988); U.S. Pat. Nos. 4,889,818 and 4,955,188), Tne DNA polymerase (WO 96/10640), Tma DNA polymerase (U.S. Pat. No. 5,374,553) and mutants, variants or derivatives thereof (see, e.g., copending U.S. patent application Ser. Nos. 08/706,702 and 08/706,706, of A. John Hughes and Deb K. Chattedee, both filed Sep. 9, 1996, which are incorporated by reference herein in their entireties). Preferred enzymes for use in the invention include those that are reduced or substantially reduced in RNase H activity. By an enzyme "substantially reduced in RNase H activity" is meant that the enzyme has less than about 20%, more preferably less than about 15%, 10% or 5%, and most preferably less than about 2%, of the RNase H activity of the corresponding wildtype or RNase H⁺ enzyme such as wildtype Moloney Murine Leukemia Virus (M-MLV), Avian Myeloblastosis Virus (AMV) or Rous Sarcoma Virus (RSV) reverse transcriptases. The RNase H activity of any enzyme may be determined by a variety of assays, such as those described, for example, in U.S. Pat. No. 5,244,797, in Kotewicz, M. L., et al., Nucl. Acids Res. 16:265 (1988) and in Gerard, G. F., et al., FOCUS 14(5):91 (1992), the disclosures of all of which are fully incorporated herein by reference. Particularly preferred such polypeptides for use in the invention include, but are not limited to, M-MLV H reverse transcriptase, RSV H⁻ reverse transcriptase, AMV H⁻ reverse transcriptase, RAV (Rous-associated virus) H- reverse transcriptase, MAV (myeloblastosis-associated virus) H- reverse transcriptase and HIV H⁻ reverse transcriptase. It will be understood by one of ordinary skill, however, that any enzyme capable of producing a DNA molecule from a ribonucleic acid molecule (i.e., having reverse transcriptase activity) that is substantially reduced in RNase H activity may be equivalently used in the compositions, methods and kits of the invention.

DNA and RNA polymerases for use in the invention may be obtained commercially, for example from QIAGEN (Hilden, Germany), Life Technologies, Inc. (Rockville, Md.), New England BioLabs (Beverly, Mass.) or ROCHE Biochemicals. Polypeptides having reverse transcriptase activity for use in the invention may be obtained commercially, for example from QIAGEN (Hilden, Germany), Life Technologies, Inc. (Rockville, Md.), Pharmacia (Piscataway, N.J.), Sigma (Saint Louis, Mo.) or ROCHE (Penzberg, Germany). Alternatively, polypeptides having reverse transcriptase activity may be isolated from their natural viral or bacterial sources according to standard procedures for isolating and purifying natural proteins that are well-known to one of ordinary skill in the art (see, e.g., Houts, G. E., et al., J. Virol. 29:517 (1979)). In addition, the polypeptides having reverse transcriptase activity may be prepared by recombinant DNA techniques that are familiar to one of ordinary skill in the art (see, e.g., Kotewicz, M. L., et al., Nucl. Acids Res. 16:265 (1988); Soltis, D. A., and Skalka, A. M., Proc. Natl. Acad. Sci. USA 85:3372-3376 (1988)).

Polypeptides having polymerase or reverse transcriptase activity are preferably used in the present compositions and methods at a final concentration in solution of about 0.1-200 units per milliliter, about 0.1-50 units per milliliter, about 0.1-40 units per milliliter, about 0.1-3.6 units per milliliter, about 0.1-34 units per milliliter, about 0.1-32 units per milliliter, about 0.1-30 units per milliliter, or about 0.1-20 units per milliliter, and most preferably at a concentration of about 20-40 units per milliliter. Of course, other suitable concentrations of such polymerases or reverse transcriptases suitable for use in the invention will be apparent to one or ordinary skill in the art and may differ in its optimal range for different polymerases.

In contrast to the disclosure in US 6,242,235 B1 it has astonishingly been found that the addition of an inert protein enables the application of ionic detergents.

In a preferred embodiment the ionic detergent is a zwitterionic detergent. Such zwitterionic detergents may be selected from the group comprising (i) 3-[(3-cholamidopropyl)dimethylammonio]-1-propanesulfonate (CHAPS), (ii) 3-[(3-cholamidopropyl)dimethylammonio]-2-hydroxy-1-propanesulfonate (CHAPSO), (iii) N-(alkyl C10-C16)-N,N-dimethylglycine betaine (EMPIGEN BB), (iv) Caprylyl sulfobetaine (SB3-10), (v) 3-[N,N-dimethyl(3-myristoylaminopropyl)ammonio]propanesulfonate (Amidosulfobetaine-14; ASB-14), (vi) N-tetradecyl-N,N-dimethyl-3-ammonio-1-propanesulfonate(3-14 Detergent; ZWITTERGENT), (vii) N-dodecyl-N,N'-dimethyl-3-ammonio-1-propanesulfonate, (viii) N-octadecyl-N,N-dimethyl-3-ammonio-1-propanesulfonate, (ix) N-decyl-N,N-dimethyl-3-ammonium-1-propanesulfonate, (x)Mirataine CB, (xi) Mirataine BB, (xii) Mirataine CBR, (xiii) Mirataine ACS, (ivx) Miracare 2MHT and, (vx) Miracare 2MCA.

Particularly preferred zwitterionic detergents are 3-[(3-cholamidopropyl)dimethylammonio]-1-propanesulfonate (CHAPS), 3-[(3-cholamidopropyl]dimethylammonio)-2-hydroxy-1-propanesulfonate (CHAPSO) and, N-(alkyl C10-C16)-N,N-dimethylglycine betaine (EMPIGEN BB).

The term "detergent" as used herein refers to amphipathic surface-active agents ("surfactants") that, when added to a liquid, reduce surface tension of the liquid in comparison to the same liquid in the absence of the detergent. See, e.g., Detergents: A guide to the properties and uses of detergents in biological systems, Calbiochem-Novabiochem Corporation, 2001, which is hereby incorporated by reference in its entirety.

The most preferred zwitterionic detergent is 3-[(3-cholamidopropyl]dimethylammonio)-2-hydroxy-1-propanesulfonate (CHAPSO).

In one embodiment the inert protein is selected from the group of inert natural or synthetic peptides, polypeptides, globulin, collagen, albumin as well as derivatives thereof, or fragments or fractions thereof. Here, the protein is preferentially present at a concentration of over 0.01 mg/ml, over 0.05 mg/ml and over 0.1 mg/ml. Ideally, the concentration is not over 2 mg/ml.

In a preferred embodiment the inert protein is bovine serum albumin (BSA) as well derivatives and fragments thereof. Fragments thereof have more than 50% of the length of naturally occurring BSA, more than 60% of the length of naturally occurring BSA, more than 70% of the length of naturally occurring BSA, more than 80% of the length of naturally occurring BSA, more than 90% of the length of naturally occurring BSA, and most preferentially more than 95% of the length of naturally occurring BSA.

In a preferred embodiment the inert protein is bovine serum albumin (BSA) and said protein is present at a concentration selected from the group of, over 0.01 mg/ml, over 0.05 mg/ml and over 0.1 mg/ml, ideally the concentration is under 2 mg/ml.

In a preferred embodiment the ionic detergent is present at a concentration of between 0.0005 % and 5.0 % by volume.

In a preferred embodiment the ionic detergent is present at a concentration of between 0.001 % and 0.4 % by volume.

It is particularly preferred that the ionic detergent is present at a concentration of between 0.002 % and 0.2 % by volume, and 0.004 % and 0.008 % by volume.

It is even more preferred that the ionic detergent is a zwitterionic detergent.

The zwitterionic detergent is preferably present at a concentration of between 0.0005 % and 5.0 % by volume.

In a preferred embodiment the zwitterionic detergent is present at a concentration of between 0.001 % and 0.4 % by volume.

It is particularly preferred that the zwitterionic detergent is present at a concentration of between 0.002 % and 0.2 % by volume, and 0.004 % and 0.008 % by volume.

The ionic detergent is preferably selected from the group of, (a) zwitterionic detergents, such as 3-[(3-cholamidopropyl)dimethylammonio]-1-propanesulfonate (CHAPS), 3-[(3-cholamidopropyl)dimethylarnmonio]-2-hydroxy-l-propanesulfonate (CHAPSO), N-(alkyl C 10-C16)-N,N-dimethylglycine betaine (EMPIGEN BB), Caprylyl sulfobetaine (SB3-10), 3-[N,N-dilnetliyl(3-myristoylaminopropyl)ammonio]propanesulfonate (Amidosulfobetaine-14; ASB-14), N-tetradecyl-N,N-dimethyl-3-ammonio-1-propanesulfonate(3-14 Detergent; ZWITTERGENT), N-dodecyl-N,N'-dimethyl-3-aminonio-1-propanesulfonate, N-octadecyl-N,N-dimethyl-3-ammonio-1-propanesulfonate, N-decyl-N,N-dimethyl-3-ammonium-1-propanesulfonate, Mirataine CB, Mirataine BB, Mirataine CBR, Mirataine ACS, Miracare 2MHT and, Miracare 2MCA, cationic detergents such as Cetylpyridinium chloride, Tetradecyl-trimethyl-ammonium bromide, Dimethyl dioctadecyl ammonium bromide and (c) anionic detergents such as Cholic acid, Taurocholic acid, Triton X-200, Triton W-30, Triton-30, Triton-770, Dioctyl sulfosuccinate.

In one embodiment the composition according to the invention is a reaction buffer and said composition additionally comprises a substance selected from the group of a buffering agent, a monovalent salt, a divalent cation and nucleotides.

The term "reaction buffer" refers to a buffering solution in which an enzymatic reaction is performed.

The term "monovalent salt" refers to any salt in which the metal (e.g., Na, K, or Li) has a net 1+ charge in solution (*i.e.,* one more proton than electron).

The term "divalent salt" refers to any salt in which a metal (e.g. Mg, Ca, Mn, or Sr) has a net 2+ charge in solution.

The term "solution" refers to an aqueous or non-aqueous mixture.

A buffering agent is preferably selected from the group of acetate buffer, sulfate buffer, phosphate buffer, MOPS, HEPES and Tris-(hydroxymethyl)aminomethane (TRIS). TRIS is most preferred.

To formulate a buffer *per sé,* a buffer salt which is preferably a salt of Tris(hydroxymethyl)aminomethane (TRIS), and most preferably the hydrochloride salt thereof, is combined with a sufficient quantity of water to yield a solution having a TRIS concentration of 5-150 mM, preferably 10-60 mM, and most preferably about 20-60 mM. To this solution, a salt of magnesium (preferably either the chloride or acetate salt thereof) may be added to provide a working concentration thereof of 1-10 mM, preferably 1.5-8.0 mM, and most preferably about 3-7.5 mM. A salt of potassium (most preferably potassium chloride) may also be added to the solution, at a working concentration of 10-100 mM and most preferably about 75 mM, A reducing agent such as dithiothreitol may be added to the solution, preferably at a final concentration of about 1-100 mM, more preferably a concentration of about 5-50 mM or about 7.5-20 mM, and most preferably at a concentration of about 10 mM. A small amount of a salt of ethylenediaminetetraacetate (EDTA), such as disodium EDTA, may also be added (preferably about 0.1 millimolar), although inclusion of EDTA does not appear to be essential to the function or stability of the compositions of the present invention. After addition of all buffers and salts, this buffered salt solution is mixed well until all salts are dissolved and the pH is adjusted using methods known in the art to a pH value of 7.4 to 9.2, preferably 8.0 to 9.0, and most preferably about 8.4.

The composition may be a storage buffer and said composition then additionally comprises a substance selected from the group of a buffering agent, a reducing agent, a chelator, a reducing agent and glycerol.

The term "storage buffer" refers to a buffering solution in which an enzyme is stored.

The terms "chelator" or "chelating agent" refer to any materials having more than one atom with a lone pair of electrons that are available to bond to a metal ion. The chelator is preferably EDTA.

The term "reducing agent" refers to material that donates electrons to a second material to reduce the oxidation state of one or more of the second material's atoms.

In one embodiment of the invention the invention relates to a method for enzymatic nucleic acid synthesis comprising the steps of (a) providing in a reaction mixture a polymerase activity, a nucleic acid template an ionic preferably, a zwitterionic detergent, a buffer, a salt, nucleotides and an inert protein stabilizer and, (b) incubating the reaction mixture at a temperature which enables nucleic acid synthesis.

As used herein, "nucleic acid" refers to both a deoxyribonucleic acid (DNA) and a ribonucleic acid (RNA), as well as modified and/or functionalized versions thereof. Similarly, the term "nucleotide" as used herein includes both individual units of ribonucleic acid and deoxyribonucleic acid as well as nucleoside and nucleotide analogs, and modified nucleotides such as labeled nucleotides. In addition, "nucleotide" includes non-naturally occurring analog structures, such as those in which the sugar, phosphate, and/or base units are absent or replaced by other chemical structures. Thus, the term "nucleotide" encompasses individual peptide nucleic acid (PNA) (Nielsen et al., Bioconjug. Chem. 1994; 5(1):3-7) and locked nucleic acid (LNA) (Braasch and Corey, Chem. Biol. 2001; 8(1):1-7)) units as well as other like units.

The method according to the invention may be selected from the group of DNA sequencing, primer extension assay, DNA amplification and reverse transcription of RNA into DNA.

The compounds and compositions of the invention maybe used in methods for the synthesis of nucleic acids. In particular, it has been discovered that the present compounds and compositions facilitate the synthesis, particularly via amplification reactions such as the polymerase chain reaction (PCR). The present compounds and compositions may therefore be used in any method requiring the synthesis of nucleic acid molecules, such as DNA (particularly cDNA) and RNA (particularly mRNA) molecules. Methods in which the compounds or compositions of the invention may advantageously be used include, but are not limited to, nucleic acid synthesis methods, nucleic acid amplification methods, nucleic acid reverse transcription methods, and nucleic acid sequencing methods.

In a preferred embodiment, the nucleic acid molecule used in the amplification method is DNA. In a preferred embodiment, the DNA molecule is double stranded. In other embodiments, the DNA molecule is single stranded. In a preferred embodiment, the double stranded DNA molecule is a linear DNA molecule. In other embodiments, the DNA molecule is non-linear, for example circular or supercoiled DNA.

In other aspects of the invention, the compositions of the invention may be used in methods for amplifying or sequencing nucleic acid molecules. Nucleic acid amplification methods according to this aspect of the invention may additionally comprise use of one or more polypeptides having reverse transcriptase activity, in methods generally known in the art as one-step (e.g., one-step RT-PCR) or two-step (e.g., two-step RT-PCR) reverse transcriptase-amplification reactions. For amplification of long nucleic acid molecules (*i.e.*, longer than about 3-5 Kb in length), the compositions of the invention may comprise a combination of polypeptides having DNA polymerase activity, as described in detail in commonly owned copending U.S. application Ser. No. 08/801,720, filed Feb. 14, 1997, the disclosure of which is incorporated herein by reference in its entirety.

Amplification methods according to this aspect of the invention may comprise one or more steps. For example, the invention provides a method for amplifying a nucleic acid molecule comprising (a) mixing a nucleic acid template with one or more of the above-described compounds or compositions to form a mixture; and (b) incubating the mixture under conditions sufficient to amplify a nucleic acid molecule complementary to all or a portion of the template. The invention also provides nucleic acid molecules amplified by such methods.

General methods for amplification and analysis of nucleic acid molecules or fragments are well-known to one of ordinary skill in the art (see, e.g., U.S. Pat. Nos. 4,583,195; 4,683,202; and 4,800,159; Innis, M. A., et al,, eds., PCR Protocols: A Guide to Methods and Applications, San Diego, Calif.: Academic Press, Inc. (1990); Griffin, H. G., and Griffin, A. M., eds., PCR Technology: Current Innovations, Boca Raton, Fla.: CRC Press (1994)). For example, amplification methods which may be used in accordance with the present invention include PCR (U.S. Pat. Nos. 4,683,195 and 4,683,202), Strand Displacement Amplification (SDA; U.S. Pat. No. 5,455,166; EP 0 684 315), and Nucleic Acid Sequence-Based Amplification (NASBA; U.S. Pat. No. 5,409,818; EP 0 329 822).

With the methods and compositions according to the invention it is possible to perform a combined amplification and sequencing reaction ('DEXAS') directly from complex DNA mixtures by using two thermostable DNA polymerases, one that favours the incorporation of deoxynucleotides over dideoxynucleotides, and one which has a decreased ability to discriminate between these two nucleotide forms. During cycles of thermal denaturation, annealing and extension, the former enzyme primarily amplifies the target sequence whereas the latter enzyme primarily performs a sequencing reaction. This method allows the determination of single-copy nuclear DNA sequences from amounts of human genomic DNA comparable to those used to amplify nucleotide sequences by the polymerase chain reaction. Thus, DNA sequences can be easily determined directly from total genomic DNA ("Direct DNA sequence determination from total genomic DNA", Kilger et al., Nucleic Acids Res. 1997 May 15; 25(10): 2032-2034)

Typically, amplification methods comprise contacting the nucleic acid sample with a compound or composition (such as those of the present invention) comprising one or more polypeptides having nucleic acid polymerase activity in the presence of one or more primer sequences, amplifying the nucleic acid sample to generate a collection of amplified nucleic acid fragments, preferably by PCR or equivalent automated amplification technique, and optionally separating the amplified nucleic acid fragments by size, preferably by gel electrophoresis, and analyzing the gels for the presence of nucleic acid fragments, for example by staining the gel with a nucleic acid-binding dye such as ethidium bromide.

Following amplification by the methods of the present invention, the amplified nucleic acid fragments may be isolated for further use or characterization. This step is usually accomplished by separation of the amplified nucleic acid fragments by size by any physical or biochemical means including gel electrophoresis, capillary electrophoresis, chromatography (including sizing, affinity and immunochromatography), density gradient centrifugation and immunoadsorption. Separation of nucleic acid fragments by gel electrophoresis is particularly preferred, as it provides a rapid and highly reproducible means of sensitive separation of a multitude of nucleic acid fragments, and permits direct, simultaneous comparison of the fragments in several samples of nucleic acids. One can extend this approach, in another preferred embodiment, to isolate and characterize these fragments or any nucleic acid fragment amplified by the methods of the invention.

In this embodiment, one or more of the amplified nucleic acid fragments are removed from the gel which was used for identification (see above), according to standard techniques such as electroelution or physical excision. The isolated unique nucleic acid fragments may then be inserted into standard nucleotide vectors, including expression vectors, suitable for transfection or transformation of a variety of prokaryotic (bacterial) or eukaryotic (yeast, plant or animal including human and other mammalian) cells. Alternatively, nucleic acid molecules that are amplified and isolated using the compounds, compositions and methods of the present invention may be further characterized, for example by sequencing (*i.e.,* determining the nucleotide sequence of the nucleic acid fragments), by methods described below and others that are standard in the art (see, e.g., U.S. Pat. Nos. 4,962,022 and 5,498,523, which are directed to methods of DNA sequencing).

Nucleic acid sequencing methods according to the invention may comprise one or more steps. For example, the invention provides a method for sequencing a nucleic acid molecule comprising (a) mixing a nucleic acid molecule to be sequenced with one or more primers, one or more of the above-described compounds or compositions of the invention, one or more nucleotides and one or more terminating agents (such as a dideoxynucleotide) to form a mixture; (b) incubating the mixture under conditions sufficient to synthesize a population of molecules complementary to all or a portion of the molecule to be sequenced; and (c) separating the population to determine the nucleotide sequence of all or a portion of the molecule to be sequenced.

Nucleic acid sequencing techniques which may employ the present compositions include dideoxy sequencing methods such as those disclosed in U.S. Pat. Nos. 4,962,022 and 5,498,523.

The invention also relates to a kit comprising a composition according to the invention. Said kit may also comprise additional reagents such as salts, primers, buffers, further enzymes and the like.

### Examples

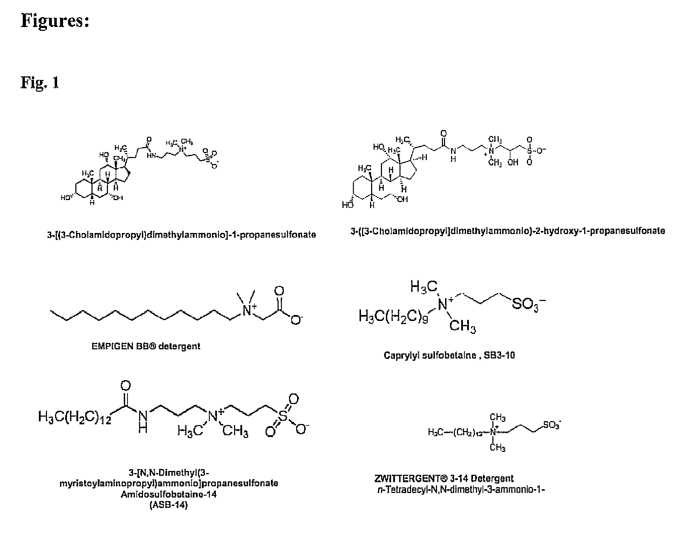

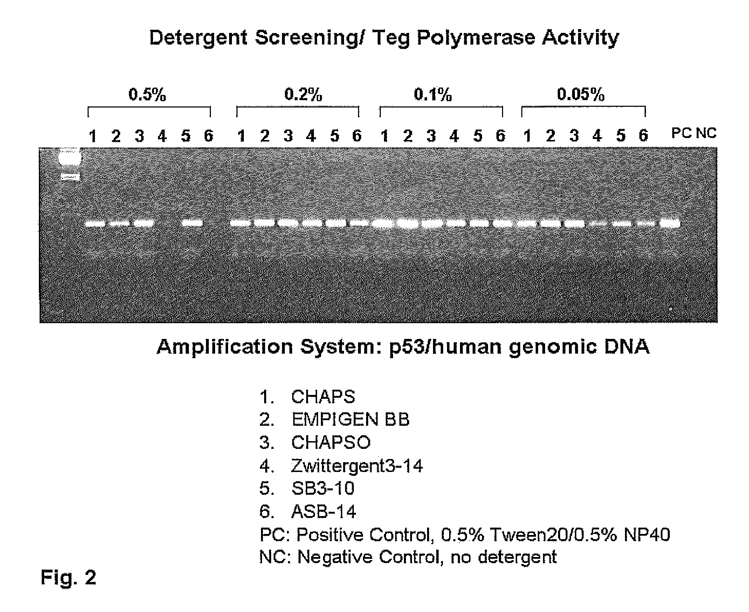

- Example 1:: Example 1 shows preferred zwitterionic detergents in figure 1.
- Example 2:: Example 2 shows that the combination of zwitterionic detergents and BSA enhances polymerase stability and enables a PCR reaction: Here detergent-free Teg DNA polymerase was diluted in storage buffers without any detergent (negative control), or with different zwitterionic detergents at different concentrations, or non-ionic detergents NP-40/Tween20 (positive control) to a final concentration of 14 ng/µl. 25 µl of PCR reaction mix was set up with a Teg PCR buffer containing 0.1 mg/ml BSA (final concentration), human genomic DNA, primers for human p53 gene, dNTPs, and 1µl Teg with different detergents. Amplification conditions are as following: 94°C for 5 minutes; followed by 35 cycles of: 94°C for 30 seconds, 60°C for 30 seconds and 72°C for 1 minute; then followed by a final elongation step: 72°C for 10 minutes. A successful PCR should generate an amplification product of about 500 bp.
- Example 3:: Example 3 shows that the addition of BSA is essential for the function of zwitterionic detergents. In a similar test as that in Figure 2, PCR reactions with PCR buffer, Teg in storage buffers combined with different zwitterionic detergents, dNTPs, human genomic DNA, primers to amplify human prp gene (750 bp), but not BSA, failed to generate any PCR products.
- Example 4:: Example 4 shows the function of zwitterionic detergents and BSA on Taq polymerase. In the PCR reactions with zwitterionic detergent CHAPSO (final concentration 0.032%) or BSA (0.1 mg/ml) alone, Taq could not amplify the target gene (human prp, 750 bp). However, the combination of BSA and CHAPSO stabilized Taq and led to successful PCR.

### Figure captions

- Figure 1:: Figure 1 shows preferred zwitterionic detergents.
- Figure 2:: Figure 2 shows that all detergents tested were able to stabilize polymerase and enhance PCR performance (evidenced by the generation of the 500 bp PCR products), albeit with different optimal concentrations. In contrast, the PCR with BSA alone but no detergent (negative control) was not able to generate any products. Several other PCR systems were also tested and gave similar results: human cyst (1.5 kb product), murine PKC (2 kb ), human prp (750 bp) (Data not shown).

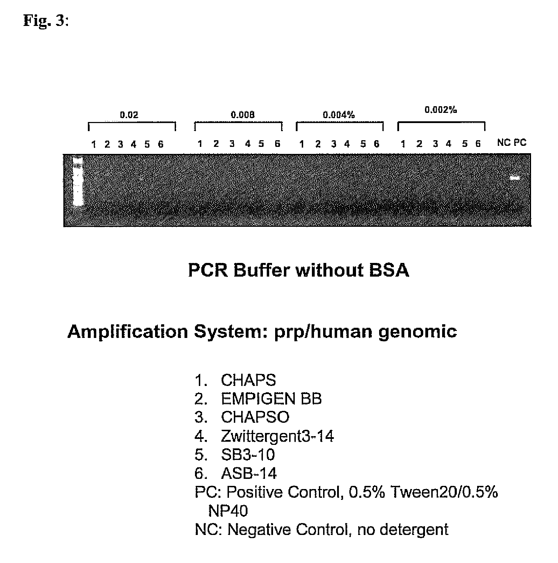

- Figure 3:: The experiment in figure 3 reproduced the observation that the combination with BSA is essential for the function of ionic, preferably zwitterionic detergents to enhance polymerase stability.

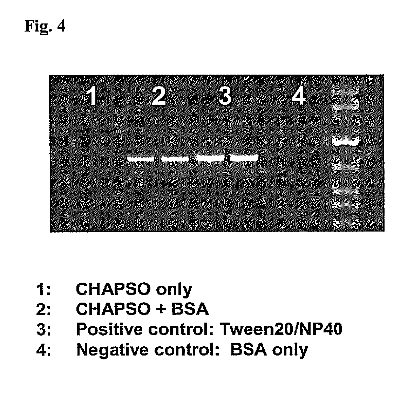

- Figure 4:: Figure 4 shows that a combination of BSA and zwitterionic detergent enhances in particular Taq polymerase activity.

## Claims

1. Composition comprising
a. an enzyme with nucleic acid polymerase activity,
b. an inert protein and,
c. an ionic detergent.

2. Composition according to claim 1, wherein the ionic detergent is a zwitterionic detergent.

3. Composition according to claim 1 or 2, wherein the inert protein is selected from the group of inert natural or synthetic peptides, polypeptides, globulin, collagen as well as derivatives thereof, and serum albumin as well derivatives and fragments thereof.

4. Composition according to claim 3, wherein the inert protein is bovine serum albumin (BSA) and said protein is present at a concentration selected from the group of, over 0.01 mg/ml, over 0.05 mg/ml and over 0.1 mg/ml.

5. Composition according to claims 1 to 4, wherein the ionic detergent is present at a concentration of between 0.0005 % and 5.0 % by volume.

6. Composition according to claim 1 to 5, wherein the ionic detergent is present at a concentration of between 0.001 % and 0.4 % by volume.

7. Composition according to claims 1, 3, 4, 5 and 6, wherein the ionic detergent is selected from the group of,
a. Zwitterionic detergents:
i. 3-[(3-cholamidopropyl)dimethylammonio]-1-propanesulfonate (CHAPS),
ii. 3-[(3-cholamidopropyl]dimethylammonio)-2-hydroxy-1-propanesulfonate (CHAPSO),
iii. N-(alkyl C10-C16)-N,N-dimethylglycine betaine (EMPIGEN BB),
iv. Caprylyl sulfobetain (SB3-10),
v. 3-[N,N-dimethyl(3-myristoylaminopropyl)ammonio]propanesulfonate (Amidosulfobetain-14; ASB-14),
vi. N-tetradecyl-N,N-dimethyl-3-ammonio-1-propanesulfonate(3-14 Detergent; ZWITTERGENT),
vii. N-dodecyl-N,N'-dimethyl-3-ammonio-1-propanesulfonate,
viii. N-octadecyl-N,N-dimethyl-3-ammonio-1-propanesulfonate,
ix. N-decyl-N,N-dimethyl-3-ammonium-1-propanesulfonate,
x. Mirataine CB,
xi. Mirataine BB,
xii. Mirataine CBR,
xiii. Mirataine ACS,
xiv. Miracare 2MHT and,
xv. Miracare 2MCA.
b. Cationic detergents:
i. Cetylpyridinium chloride
ii. Tetradecyl-trimethyl-ammonium bromide
iii. Dimethyl dioctadecyl Ammonium bromide
c. Anionic detergents
i. Cholic acid
ii. Taurocholic acid
iii. Triton X-200
iv. Triton W-30
v. Triton X-301
vi. Triton 770
vii. Dioctyl sulfosuccinate

8. Composition according to claim 7, wherein the zwitterionic detergent is selected from the group of,
a. 3-[(3-cholamidopropyl)dimethylammonio]-1-propanesulfonate (CHAPS),
b. 3-[(3-cholamidopropyl)dimethylammonio]-2-hydroxy-1-propanesulfonate (CHAPSO) and,
c. N-(alkyl C10-C16)-N,N-dimethylglycine betaine (EMPIGEN BB).

9. Composition according to any of the preceding claims 1 to 8, wherein the composition is a reaction buffer and said composition additionally comprises a substance selected from the group of, a buffering agent, a monovalent salt, a divalent cation and nucleotides.

10. Composition according to any of the claims 1 to 8, wherein the composition is a storage buffer and said composition additionally comprises a substance selected from the group of, a buffering agent, a reducing agent, a chelator, a reducing agent and glycerol.

11. Composition according to claim 9 or 10, wherein the buffering agent is selected from the group of acetate buffer, sulfate buffer, phosphate buffer, MOPS, HEPES and Tris-(hydroxymethyl)aminomethane (TRIS).

12. Method for enzymatic nucleic acid synthesis comprising the steps of,
a. providing in a reaction mixture, a polymerase activity, a nucleic acid template, a ionic detergent, preferably zwitterionic detergent, a buffer, a salt, nucleotides and an inert protein and,
b. incubating the reaction mixture at a temperature which enables nucleic acid synthesis.

13. Method according to claim 12, wherein the enzymatic nucleic acid synthesis is performed in a method selected from the group of, DNA sequencing, primer extension assay, DNA amplification and reverse transcription of RNA into DNA.

14. Kit comprising a composition according to any of the claims 1 to 11 and/or for performing the method of claims 12 to 13.
